Europäisches Patentamt

European Patent Office

Office européen des brevets

(11) Publication number: **0 331 345**
**A2**

## EUROPEAN PATENT APPLICATION

(21) Application number: 89301744.2

(51) Int. Cl.4: **A61F 2/24**

(22) Date of filing: **23.02.89**

(30) Priority: **02.03.88 US 162924**

(43) Date of publication of application:
**06.09.89 Bulletin 89/36**

(84) Designated Contracting States:
**AT CH DE FR GB IT LI**

(71) Applicant: **Pfizer Hospital Products Group, Inc.**
**235 East 42nd Street**
**New York New York 10017(US)**

(72) Inventor: **Silvestrini, Thomas A.**
**12, Naomi Lane**
**East Lyme Connecticut(US)**

(74) Representative: **Moore, James William, Dr. et al**
**Pfizer Limited Ramsgate Road**
**Sandwich Kent CT13 9NJ(GB)**

(54) **Triaxially woven fabric for heart valve.**

(57) A heart valve prosthesis has leaflets made of a triaxially woven fabric having three angularly displaced sets of substantially parallel courses of yarn interwoven to prevent slippage of at least one yarn course along any other yarn course. The fabric is preferably embedded in a polymeric matrix.

EP 0 331 345 A2

FIG. 2

## TRIAXIALLY WOVEN FABRIC FOR HEART VALUE

The invention relates to a heart valve prosthesis having leaflets made of a triaxially woven fabric.

Heart valve prostheses are widely used to replace natural heart valves in patients suffering from heart disease. Such heart valve prostheses may comprise leaflets made of a variety of tissue materials or synthetic fabrics. Suitable natural tissues are for example disclosed in U.S. Patent Nos. 3,966,401 and 4,323,358 and suitable synthetic fabrics in U.S. Patent Nos. 4,192,020, 4,340,091 and 4,610,688.

The heart leaflet fabrics must satisfy many conditions. They must have mechanical properties resembling those of natural leaflet tissue, and they must be biocompatible and resistant to blood clotting and mineralization. There is therefore a constant search for new fabrics with improved properties.

According to the invention, a heart valve prosthesis is provided comprising a frame having a generally circular base defining the circumferential direction of the prosthesis, and means extending from said base and defining the axial direction of the prosthesis, said means supporting a plurality of triaxially woven fabric elements such that said elements function as heart valve leaflets during operation of the valve, said fabric having three angularly displaced sets of substantially parallel courses of yarn interwoven to prevent slippage of at least one yarn course along any other yarn course. The triaxially woven fabric of use in the invention is described in U.S. Reissue Patent 28,155.

Figure 1 shows a heart valve in perspective view.

Figure 2 is a sheet in which only a portion of the triaxial weave is shown.

Figure 3 is the sheet of figure 2 formed into a cylinder.

Figure 4 is the cylinder of figure 3 formed into a three leaflet heart valve shape.

Figure 5 is a heart valve leaflet formed from the cylinder of figure 3 wherein one yarn course c is oriented in the circumferential direction and two yarn courses r are oriented substantially at 90 degrees to all edges of the heart valve frame.

Figure 6 is one embodiment of a biplain triaxial weave.

The heart valve prosthesis of use in the invention may be any prior art valve capable of supporting leaflet tissues made of natural or synthetic materials. The prosthesis of the invention comprises a frame having a generally circular base defining the circumferential direction of the prosthesis. The frame is made of a biocompatible synthetic, ceramic or metal material. Synthetic frame materials include pyrolytic carbon, and polymeric materials such as Delrin which is a polyacetal resin, polyolefins, and softer polymeric materials such as foamed polyurethane. Although the frame obviously must have some stiffness to allow for free blood flow, materials softer than metal are suitable as frame material. Examples of suitable biocompatible metals are titanium and cobalt-chromium-molybdenum alloy such as Vitallium®.

The means extending from the generally circular base and defining the axial direction of the prosthesis may comprise a plurality of spaced members. Suitable heart valve prostheses having three such spaced members supporting three heart valve leaflets are for instance disclosed in U.S. Patents 4,629,459, 4,340,091 and 4,191,218. Bileaflet valves are also suitable in practicing the invention.

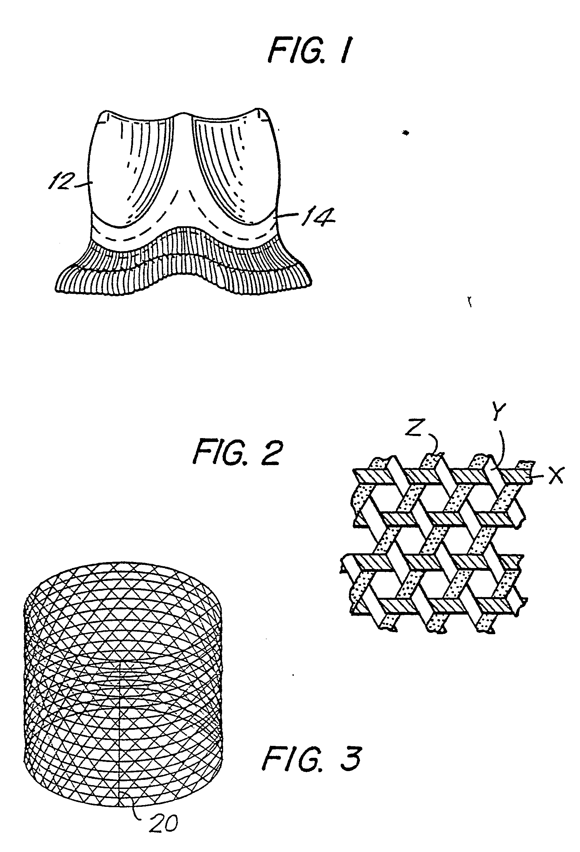

Figure 1 illustrates one specific embodiment of a suitable heart valve prosthesis as disclosed in above U.S. Patent 4,629,459. The prosthesis has three heart valve tissue leaflets 12, a stent assembly 14 having a plurality (three) of spaced members 16 and a suture or sewing ring frame 18.

The three heart valve leaflets 12 are made of a triaxially woven fabric, an embodiment of which is shown in figure 2, as part of a sheet of such fabric. The fabric has three yarn courses x, y and z. The x yarn courses are woven over the y yarn courses and under the z yarn courses, the y yarn courses are woven over the z yarn courses and under the x yarn courses, and the z yarn courses are woven over x and under y. The embodiment in figure 2 is the most simple triaxially woven fabric. Many variations are feasible as disclosed in above Reissue 28,155.

In figure 3, the edges of the sheet in figure 2 are brought together and kept in place by known techniques such as sewing, or ultrasonic welding in the case of a synthetic fabric. Utrasonic welding results in seam 20.

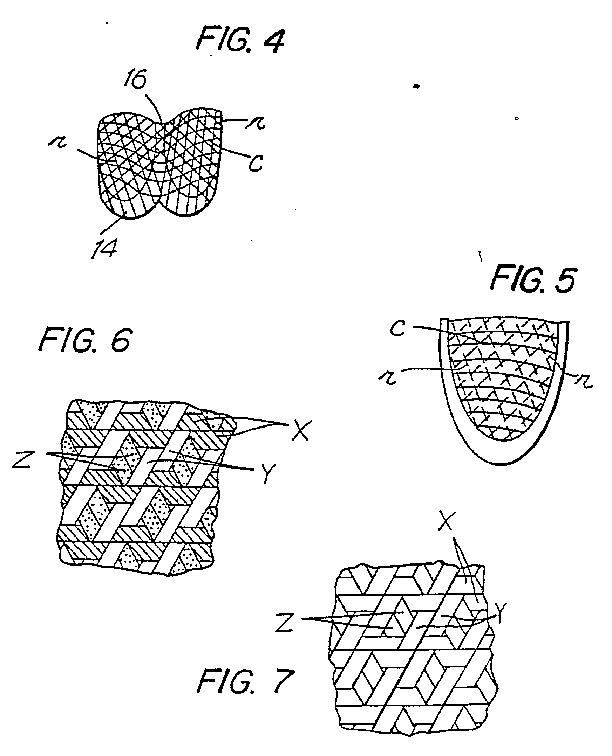

Figure 4 shows the cylinder of figure 3 after draping over spaced members 16 of stent 14 and shaping in the form of heart valve leaves which will be attached by known means to members 16 and stent 14.

Preferably, the cylinder is draped over the heart valve prosthesis such that one set of parallel yarn courses is oriented substantially in the circumferential direction c of the prosthesis and the other two sets of yarn courses are oriented in radial directions r at nearly 90° to all edges of the heart valve member 16. Such orientation results in a heart valve leaflet having more stretch in the radial direction than in the circumferential direction. This two way stretch is important to heart valve leaflet construction since in this manner the fabric is flexible in the flow direction allowing the leaflets to bend open easily for low pressure drop through the valve, whereas the fabric is stiff in the circumferential direction strengthening the leaflets to resist the strain of blood flow through the valve once the leaflets are open.

The two way stretch can be enhanced by using a bicomponent triaxially woven fabric wherein one yarn set is made of one particular material and the other two yarn sets are made of a different material. Advantageously, a high strength material is used for orientation in the circumferential direction and an elastomeric material is used for orientation in the two radial directions.

In general, the material of the yarns may be a natural material such as silk, an inorganic material such as ceramic, silicon carbide or stainless steel, or a synthetic material such as carbon or synthetic polymers. Examples of suitable high strength fibers are crystalline, highly oriented fibers such as Dacron®, which is the polyester polyethylene terephthalate, ultra high modulus fibers such as Kevlar®, which is an aromatic polyamide, carbon fibers, ultradrawn polyolefins, liquid crystal polymers such as Vectran®, which is an aromatic polyester, and fibers made of polyether ether ketone or polysulfone.

Examples of suitable elastomeric fiber materials are any biocompatible elastomeric polymers or block copolymers, for example polysiloxane, polyurethane such as spandex,. C-Flex which is an olefinic polymer blend, hard elastic polyolefin, natural or synthetic rubber, or Hytrel® which is a polyester/polyether block copolymer.

Suitable combinations of high strength materials and elastomeric materials are, for example, Hytrel® and Dacron®, or Hytrel® and ultradrawn polyethylene.

The triaxially woven fabrics of the invention may have different area porosities ranging within broad limits, e.g. from less than 1% for tightly woven fabrics to about 33 to 66% for open weave fabrics. By area porosity is understood the area of the fabric pores divided by the entire area of the fabric, multiplied by 100. Tightly woven fabrics generally are stronger, whereas open weave fabrics allow for growth of new natural tissue in and around the pores of the fabric.

The porosity of a fabric depends on a number of factors. One factor is the type of triaxial weave construction as extensively disclosed in above Reissue 28,155. One weave construction not specifically disclosed in the Reissue patent is the biplain triaxial weave, embodiments of which are shown in figures 6 and 7. The embodiment in figure 6 has horizontal yarn courses x woven both over and under two sets of yarn courses y and z. In figure 7, two sets of two yarn courses x and y, out of the total of three yarn courses, are woven over and under two sets of the remaining yarn course z. Other biplain weave constructions will be apparent to those skilled in the weaving art. Generally, the biplain weaves provide lower porosity and therefore greater dimensional stability.

Porosity is further determined by the size of the yarns in each of the three sets of yarn courses in a triaxial fabric. A wide range of fiber diameter may be employed, e.g. from 5 denier (0.024 mm at 1.2 g/cm$^3$ density) to 500 denier (0.28 mm at 0.9 g/cm$^3$ density), usually from 30 (0.065 mm at 1.0 g/cm$^3$ density) to 70 denier (about 0.1 mm at 1.2 g/cm$^3$ density), depending to a large extent on the fiber material used. Usually, very fine fibers require a high strength material and fibers of larger diameter are made of a low strength elastomeric material. Accordingly, tightly woven fabrics are usually made of relatively high strength materials whereas open weave fabrics are usually made of elastomeric materials.

For added strength, the open weave fabrics may be embedded in a matrix of a polymer. Conveniently, such polymer is an elastomeric polymer of the types disclosed above with respect to fiber materials of use in the present triaxially woven fabrics. Among these elastomeric polymers, polyurethane is a preferred polymeric matrix. For application of the matrix, any solvent capable of dissolving the particular elastomeric polymer may be employed, in general. The choice of a particular solvent depends on many variables such as the desired concentration of the polymer in the solvent and the molecular weight of the polymer or mixture of polymers used. Alternatively, thermoplastic elastomers may be applied in a melt without a solvent. In any event, reinforcement of heart valve leaflets with a polymeric matrix is known so that those skilled in the heart valve leaflet art can determine the proper application conditions.

The diameter size of a yarn also depends on whether the triaxial weave is monocomponent, bicomponent or tricomponent. In a monocomponent triaxial weave, the three sets of yarn courses are all made of the same material. In a bicomponent weave, as mentioned above, two of the three sets of yarn courses are made of different materials. All three sets of yarn courses are made of a different material in a three component weave. It should be clear that a monocomponent weave of elastomeric fibers may require larger fiber diameters for necessary strength than a monocomponent weave of high strength fibers.

The following example illustrates the invention.

## EXAMPLE

A triaxially woven Dacron fabric of 440 denier yarns supplied by Gentex Inc. was cut with an ultrasonic trim knife into one inch (2.54 cm) wide by 5 inch (12.7 cm) long pieces of fabric with fibers x of Fig. 1 oriented in the long (5 inch) direction. The ultrasonic knife sealed the fabric along the cuts making the fabric more stable to handle and preventing unraveling at the edges. The cut piece of fabric was welded into a cylinder (Fig. 3) by inserting the fabric into a cutting template and joining the short edges of the fabric by welding.

The fabric cylinder was slipped over a 29 mm Ionescu-Shiley valve frame of the type shown in Figure 1 supplied by Shiley Inc. (Irvine, California) such that the seam of the cylinder aligned with one of the valve members 16. The fabric cylinder was pinched with homostats to shape the cylinder into coapting valve leaflets (Fig. 4) on the valve frame. The exposed parts of the fabric leaflets were painted with solutions of 6% by weight polyurethane, supplied under the name Pellethane®, in dimethylformamide solvent, and were allowed to dry under ambient conditions. After application of two layers of polyurethane, the hemostats were removed and the entire surfaces of fabric and valve frame were coated with successive layers of polyurethane by painting and drying steps.

The resulting valves were completed by sewing on the sewing ring frame (18 in Fig. 1) and by sewing the fabric to the valve members (16 in Fig. 1).

## Claims

1. A heart valve prosthesis comprising a frame having a generally circular base defining the circumferential direction of the prosthesis, and means extending from said base and defining the axial direction of the prosthesis, characterized in that said means supports a plurality of triaxially woven fabric elements such that said elements function as heart valve leaflets during operation of the valve, said fabric having three angularly displaced sets of substantially parallel courses of yarn interwoven to prevent slippage of at least one yarn course along any other yarn course.

2. A prosthesis according to claim 1 or 2, characterized in that said fabric of elastomeric yarns is embedded in a polymeric matrix.

3. A prosthesis according to claim 1 characterized in that said courses of yarn are made of the same material, one of said courses of yarn being oriented in said circumferential direction of said frame.

4. A prosthesis according to claim 1 or 2, characterized in that said yarns are made from elastomeric material.

5. A prosthesis according to claim 1 or 2, characterized in that one of said courses of yarn is made of a material different from said other two courses of yarn.

6. A prosthesis according to claim 5, characterized in that one of said courses of yarn is oriented in the circumferential direction and is made from high strength yarn, and said two remaining courses of yarn are made of elastomeric yarn.

7. A prosthesis according to claim 6, characterized in that said high strength yarn is made of polyester, and said elastomeric yarn is made of polyurethane.

8. A prosthesis according to claim 1, characterized in that each of said courses of yarn is made of a different material.

9. A prosthesis according to any one of claims 1 to 8, characterized in that said heart valve leaflets are made of one sheet of said fabric.

10. A prosthesis according to claim 9, characterized in that said sheet is in the form of a cylinder draped over said means, which means comprises three spaced members.

## FIG. 1

12

14

## FIG. 2

Z  Y

X

## FIG. 3

20

FIG. 4

FIG. 5

FIG. 6

FIG. 7